# EUROPEAN PATENT SPECIFICATION

(11) **EP 3 391 855 B1**
(45) Date of publication and mention of the grant of the patent: **27.05.2020**
(21) Application number: 16875667.4
(22) Date of filing: 14.12.2016
(51) Int. Cl.: A61F 2/16

(54) **LENS HOLDER AND INJECTOR FOR INTRAOCULAR LENS**
LINSENHALTERUNG UND INJEKTOR FÜR INTRAOKULARLINSE
PORTE-LENTILLE ET INJECTEUR DESTINÉ À UNE LENTILLE INTRAOCULAIRE

(30) Priority: 15.12.2015 JP 2015244061
(43) Date of publication of application: 24.10.2018
(73) Proprietor: Santen Pharmaceutical Co., Ltd., Higashiyodogawa-ku Osaka-shi Osaka 533-8651 (JP)
(72) Inventor: MATSUMOTO, Kazuma, Osaka-shi Osaka 533-8651 (JP); TANAKA, Takashi, Osaka-shi Osaka 533-8651 (JP)
(74) Representative: Müller-Boré & Partner Patentanwälte PartG mbB
(86) International application number: PCT/JP2016/087169
(87) International publication number: WO 2017/104690

(56) References cited:
- EP-A1- 2 055 265
- EP-A1- 2 074 962
- JP-A- 2009 112 355
- JP-A- 2010 273 985
- JP-A- 2010 273 985
- JP-A- 2012 125 355
- JP-A- 2012 512 712
- US-A1- 2010 160 926

## Description

### TECHNICAL FIELD

The present invention relates to a structure of a lens holder of an intraocular lens injector.

### BACKGROUND ART

Conventionally, in cataract surgery in which a clouded crystalline lens in the eye is replaced with an artificial intraocular lens, an intraocular lens injector has been used that delivers an intraocular lens set in a lens holder into the eye by pushing a plunger that was inserted into a main body. With the intraocular lens injector, since there is risk of insertion of the intraocular lens not being appropriately performed if contacting with the intraocular lens in a state in which the leading end of the plunger is deviating from the appropriate position, a shaft shake prevention structure may be provided for controlling the posture of the plunger.

Patent Documents 1 to 3 are publications disclosing this type of intraocular lens injector. Patent Document 1 discloses a structure that prevents shaft shake in the left/right direction of the plunger by way of a shaft projecting part formed in a pair of rod shapes formed from a ceiling to the bottom of a lens holder. Patent Document 2 discloses a configuration of the insertion instrument of the intraocular lens in which an abutting force exerted on a sliding contact portion is gradually increased as the intraocular lens is gradually exposed from an inclined opening. Patent Document 3 discloses a configuration of insertion instrument of the intraocular lens which further includes resistance increasing means for increasing a pushing resistance of a plunger to an instrument main body when the intraocular lens is released into the eyeball from a leading end of an insertion tube portion or after the release.
Patent Document 1: Japanese Unexamined Patent Application, Publication No. 2010-273985
Patent Document 2: Japanese Unexamined Patent Application, Publication No. 2009-160151
Patent Document 3: PCT International Publication No. WO13/137208 Further relevant prior art is disclosed by US 2010/160926 A1, JP2010273985A and EP2055265A1.

### DISCLOSURE OF THE INVENTION

### Problems to be Solved by the Invention

The intraocular lens injector is configured so as to narrow as approaching towards a leading-end side for releasing into the eye by folding an optical part of the intraocular lens during the course of insertion. From the viewpoint of avoiding interference with the inner wall on the leading-end side of the intraocular lens injector, the leading-end side of the plunger is preferably formed to be thin. However, in the case of forming the leading-end side of the plunger to be thin, since an opening at the outlet of the main body must be formed according to the shape on the base-end side, which is thicker than the leading-end side, the gap between the leading-end side of the plunger and the opening becomes larger, and there is a risk of shaft shake occurring at the leading-end side of the plunger.

In this respect, in the configuration disclosed in Patent Document 1, when using a plunger having a tapered width at the leading-end side, a gap increases between the plunger at an initial position and a pair of axially exiting portions. Also, with this configuration, it is difficult to suppress the upward shifting of the plunger inside the lens holder.

Further, in the configuration disclosed in Patent Document 2, as the intraocular lens is gradually exposed from the inclined opening, since the abutting force exerted on the sliding contact portion is gradually increased, it is not possible to effectively prevent the shaft shake when the plunger comes into contact with the intraocular lens. Even in the configuration disclosed in Patent Document 3, since the pushing resistance of the plunger into the instrument main body is increased when the intraocular lens is released into the eyeball from the leading end of the insertion tube portion or after the release, it is not possible to prevent the shaft shake when the plunger comes into contact with the intraocular lens. As described above, the related art has room for improvement from the viewpoint of preventing the shaft shake inside the lens holder.

An object of the present invention is to provide a configuration of a lens holder of an intraocular lens injector which can effectively prevent the shaft shake of the plunger inside the lens holder.

### Means for Solving the Problems

According to an aspect of the present invention, there is provided a lens holder of an intraocular lens injector which extrudes the intraocular lens by a plunger and inserts the intraocular lens into an eye, the lens holder including: a holder main body which has a mount unit for setting a lens main body of the intraocular lens and in which an insertion hole of the plunger for extruding the lens main body set on the mount unit to the leading-end side is formed on a base-end side; and an elastic member which is provided in a movement path of the plunger between the mount unit and the insertion hole inside the holder main body and deforms in conformance with the shape of the plunger when the plunger moves to the leading-end side.

The elastic member preferably has a first restriction member which protrudes from a surface of the holder main body on which the mount unit is disposed and is formed on one side in a direction orthogonal to a movement direction of the plunger; and a second restriction member which protrudes from a surface of the holder main body on which the mount unit is disposed and is formed on the other side in a direction orthogonal to the movement direction of the plunger, and the first restriction member and the second restriction member are preferably formed to approach each other as the first restriction member and the second restriction member go away from the surface on which the mount unit is disposed, and are disposed to sandwich the plunger in the direction orthogonal to the movement direction.

The elastic member preferably further has a first wall part which is disposed on a leading-end side of the first restriction member and has a surface facing the leading-end side; and a second wall part which is disposed on the leading-end side of the second restriction member and has a surface facing the leading-end side.

The elastic member preferably protrudes from a surface opposite to the surface of the holder main body on which the mount unit is disposed, and preferably comes into contact with the plunger in a state in which the leading end thereof is bent to one side or the other side in the movement direction of the plunger.

The lens holder preferably further includes a lower part side support member which is provided in the movement path of the plunger between the mount unit and the insertion hole, and protrudes from a lower part of the lens holder in a direction orthogonal to the movement direction of the plunger.

According to another aspect of the present invention, there is provided an intraocular lens injector which includes a main body formed in a tubular shape; a lens holder in which the intraocular lens is accommodated, the lens holder being disposed at a leading end of the main body; and a plunger which is inserted into the main body to extrude the intraocular lens set on the lens holder from an opening formed in a leading-end side of the main body to the leading-end side, in which the lens holder has a holder main body which has a mount unit for setting the lens main body of the intraocular lens and in which an insertion hole of the plunger for extruding the lens main body set on the mount unit to the leading-end side is formed on the base-end side, and an elastic member that is provided in the movement path of the plunger between the mount unit and the insertion hole inside the holder main body, and deforms in conformance with the shape of the plunger when the plunger moves to the leading-end side.

The plunger preferably has a leading-end part having a contact portion that comes into contact with the intraocular lens, a flat part that is connected to the base-end side of the leading-end part and is formed flat to be wider than the leading-end part, and a restricted part that is formed along an axial direction of the plunger from the leading-end part to the flat part and protrudes from the flat surface at the flat part, in which the opening has a central opening opened in accordance with the shape of the flat part, and a restriction part formed to protrude from an end face of the central opening in a thickness direction of the flat part in accordance with the shape of the restricted part, and a length of the restricted part in the axial direction is preferably set so that at least the restriction part restricts the portion from the initial position before the contact portion comes into contact with the intraocular lens until the contact portion comes into contact with the intraocular lens.

### Effects of the Invention

According to the lens holder of the intraocular lens injector of the present invention, it is possible to effectively prevent the shaft shake of the plunger inside the lens holder.

### BRIEF DESCRIPTION OF THE DRAWINGS

Fig. 1 is a perspective view illustrating an appearance of an intraocular lens injector according to a first embodiment.
Fig. 2 is a plan view illustrating a state in which an intraocular lens is set in the lens holder of the first embodiment.
Fig. 3 is a cross-sectional view illustrating the interior of the lens holder of the first embodiment as seen from the leading-end tip side.
Fig. 4 is a perspective view illustrating the aspect of the interior of the lens holder of the first embodiment in a state in which the plunger moves from the initial position to the leading-end side.
Fig. 5 is a perspective view illustrating a state in which an intraocular lens is set in the lens holder of a second embodiment.
Fig. 6 is a side sectional view illustrating an aspect of the interior of the lens holder of the second embodiment.
Fig. 7 is a perspective view illustrating a positional relation between an opening of a main body of an intraocular lens injector and the plunger at the initial position of a third embodiment.
Fig. 8 is a perspective view illustrating an aspect in which the plunger moves from the initial position toward the leading-end side in the intraocular lens injector of the third embodiment.
Fig. 9 is a perspective view illustrating an aspect of the interior of the lens holder of a fourth embodiment.
Fig. 10 is a plan view illustrating an aspect of the interior of the lens holder of the fourth embodiment.
Fig. 11 is an enlarged perspective view illustrating an axial-shift-preventing unit and its vicinity of the lens holder of the fourth embodiment.
Fig. 12 is a cross-sectional view illustrating the interior of the lens holder of the fourth embodiment as seen from the leading-end tip side.

### PREFERRED MODE FOR CARRYING OUT THE INVENTION

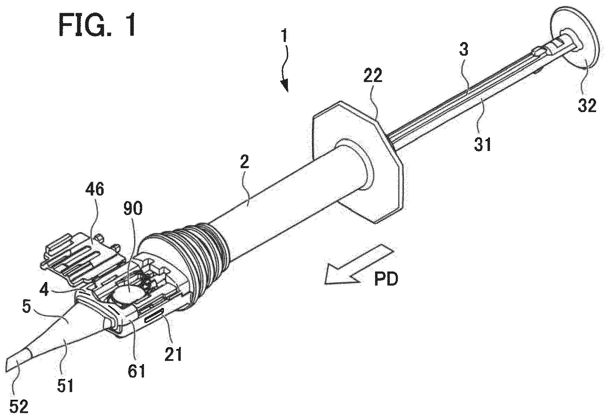

Hereinafter, each preferred embodiment of the present invention will be explained while referencing the drawings. FIG. 1 is a perspective view showing an external appearance of an intraocular lens injector 1 of a first embodiment.

The intraocular lens injector 1 shown in FIG. 1 is an insertion tool for an intraocular lens 90 that intraocularly releases the intraocular lens 90 set in the lens holder 4 from a leading-end tip 5 by pressing a plunger 3. In Fig. 1, a state in which a cover 46 of a lens holder 4 is opened is illustrated.

First, an overall configuration of the intraocular lens injector 1 will be described. As illustrated in Fig. 1, the intraocular lens injector 1 includes a main body 2, a plunger 3, a lens holder 4, and a leading-end tip 5. In the following description, the same direction as a movement direction of the plunger 3 is defined as a direction PD, and a side on which the intraocular lens is released in the direction PD is defined as a leading-end side. Further, a side opposite to the leading-end side in the direction PD is defined as a base-end side. Further, a vertical direction means the same direction as a thickness direction of the lens holder 4, and a left/right direction means a direction orthogonal to the vertical direction when the lens holder 4 is viewed in the direction PD.

The main body 2 is formed in a cylindrical shape having a through-hole for inserting the plunger 3. A plate-shaped fixing part 21 for fixing the lens holder 4 projects at an end face on the leading-end side of the main body 2 to the leading-end side. On the other hand, a brim part 22 serving as a location at which the user puts a hand is formed at an outer circumferential face on the base end side of the main body 2.

The plunger 3 is a shaft-like member that pushes the intraocular lens 90 set in the lens holder 4 to the leading-end side. The plunger 3 of the present embodiment is formed so that the diameter becomes smaller step-wise as approaching from the base side to the leading-end side.

A collar-shaped pressing part 32 is formed at the base end of the base-end side shaft part 31 located on the most base-end side of the plunger 3. The user pushes the pressing part 32 toward the main body 2 to move the plunger 3 to the leading-end side.

The lens holder 4 is for setting the intraocular lens 90 in the intraocular lens injector 1, and is arranged between the main body 2 and the leading-end tip 5. The intraocular lens 90 moves from the lens holder 4 to the leading-end tip 5 by pushing the plunger 3 toward the leading-end tip 5 side in a state in which the intraocular lens 90 is set in the lens holder 4. The detailed configuration of the lens holder 4 will be described later.

The leading-end tip 5 includes a nozzle part 51 and a release part 52. The nozzle part 51 has an internal passage communicating with the lens holder 4, and the internal passage is configured to be narrow as it goes to the leading-end side. The release part 52 is a release port that discharges the intraocular lens 90 to the outside of the intraocular lens injector 1, and is located at the leading end of the intraocular lens injector 1.

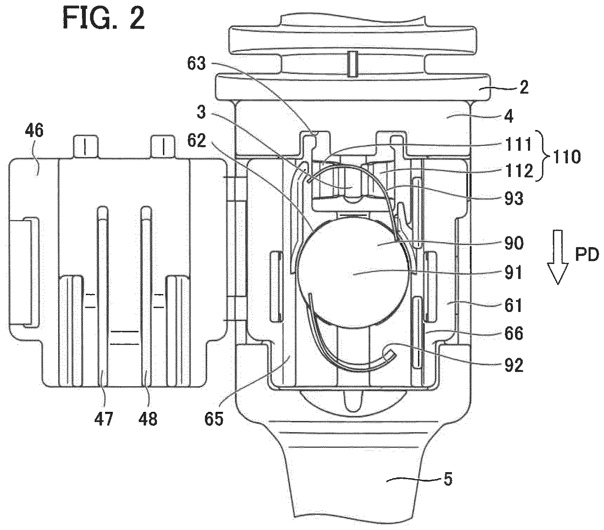

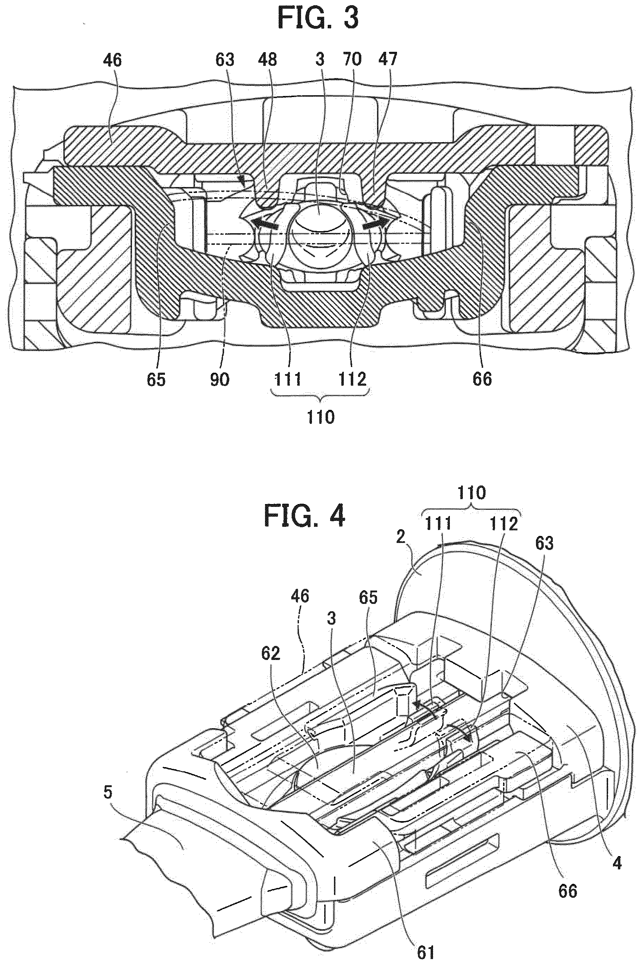

Next, the detailed configuration of the lens holder 4 will be described. Fig. 2 is a plan view illustrating a state in which the intraocular lens 90 is set in the lens holder 4. Fig. 3 is a cross-sectional view illustrating the interior of the lens holder 4 of the first embodiment as seen from the leading-end tip 5 side.

First, the intraocular lens 90 accommodated in the lens holder 4 will be described. As illustrated in Fig. 2, the intraocular lens 90 is a three-piece lens which includes an optical part 91 as a lens main body, a first lens support part 92, and a second lens support part 93. In the following description, a three-piece lens will be described as an example. However, the present invention is not limited to a three-piece lens, but can be applied to various intraocular lenses such as a single piece.

The configuration of the intraocular lens 90 will be described. The optical part 91 is a lens main body which functions as a crystalline lens after insertion into the eye. The first lens support part 92 and the second lens support part 93 are formed to extend in a curved shape from the optical part 91, and hold the optical part 91 in the eye after insertion in the eye. The first lens support part 92 and second lens support part 93 are arranged as to be in a point symmetrical positional relationship with the center of the optical part 91 as the center of symmetry. This optical part 91, first lens support part 92 and second lens support part 93 are formed to be deformable from materials having flexibility.

Next, the configuration of the lens holder 4 will be described. The lens holder 4 includes a holder main body 61, a cover 46, and an axial-shift-preventing unit 110.

The holder main body 61 is formed in a flat plate shape. The holder main body 61 has a mount unit 62 on which the optical part 91 of the intraocular lens 90 is mounted at the center of its planar part. Wall parts 65 and 66 are formed on both sides of the mount unit 62 along the direction PD, respectively. The movement of the intraocular lens 90 in a direction orthogonal to the direction PD is restricted by the wall parts 65 and 66.

As illustrated in Fig. 3, an opening 70 through which the plunger 3 is inserted is formed on the base-end side of the main body 2. An insertion hole 63 communicating with the opening 70 is formed on the base-end side of the holder main body 61. The insertion hole 63 of the present embodiment is formed by opening the end face on the base-end side of the holder main body 61.

The cover 46 is attached to the holder main body 61 so as to be openable and closable, and the inside and the outside of the lens holder 4 are isolated by closing the cover 46. Cover ribs 47 and 48 are formed on the inner face of the cover 46 along the direction PD.

The axial-shift-preventing unit 110 is a member for preventing the shaft shake of the plunger 3, and is provided inside the lens holder 4. The axial-shift-preventing unit 110 is disposed between the mount unit 62 on which the optical part 91 is mounted and the insertion hole 63.

The configuration of the axial-shift-preventing unit 110 will be described. The axial-shift-preventing unit 110 of the first embodiment includes a pair of right and left first restriction member 111 and second restriction member 112.

As illustrated in Fig. 3, the first restriction member 111 and the second restriction member 112 protrude from the lower part of the holder main body 61 toward the cover 46 in the closed state. The first restriction member 111 is disposed on one side in the left-right direction (the direction orthogonal to the movement direction) in the movement path of the plunger 3, and the second restriction member 112 is disposed on the other side in the left-right direction of the movement path of the plunger 3.

Both of the first restriction member 111 and the second restriction member 112 are inclined to approach the center in the left-right direction of the lens holder 4 as approaching the cover 46 from the lower part of the lens holder 4, and the leading end of the plunger 3 of the initial position is in a state of being sandwiched in the left-right direction. In the present embodiment, the first restriction member 111 and the second restriction member 112 have a shape corresponding to the curved surface of the plunger 3, and the first restriction member 111 and the second restriction member 112 are covered at the leading end of the cylindrical plunger 3.

Further, the first restriction member 111 and the second restriction member 112 are formed of a material that is elastically deformable to spread to the left and right sides as the plunger 3 moves. As a material which forms the first restriction member 111 and the second restriction member 112, it is preferable to use a material having a low sliding resistance, for example, polypropylene, polyethylene, nylon, polyurethane, and ABS resin so as not to hinder the movement of the plunger 3 in the movement direction more than necessary.

Further, the axial-shift-preventing unit 110 may be disposed to come into contact with the plunger 3 of the initial position in advance or may be disposed to make contact after the plunger 3 moves from the initial position. In the present embodiment, the case where the axial-shift-preventing unit 110 is molded integrally with the holder main body 61 has been described as an example, but the axial-shift-preventing unit may be incorporated separately in the holder main body.

Next, the shaft shake preventing function of the axial-shift-preventing unit 110 in the process of inserting the plunger 3 will be described. Fig. 4 is a perspective view illustrating the aspect of the interior of the lens holder 4 of the first embodiment in a state in which the plunger 3 moves from the initial position to the leading-end side. In Fig. 4, the cover 46 is illustrated by a chain line, and the state in which the intraocular lens 90 is sent to the interior of the leading-end tip 5 is illustrated.

When the plunger 3 of the initial position illustrated in Fig. 2 is pushed toward the leading-end tip 5 side, the part of the base-end side of the plunger 3 enters the inside of the lens holder 4 as illustrated in Fig. 4. At this time, since the leading end of the plunger 3 is sandwiched between the first restriction member 111 and the second restriction member 112 and the urging force is exerted, it is possible to bring the leading end of the plunger 3 into contact with the end face of the optical part 91 of the intraocular lens 90 at the appropriate position, without causing the shift until contact with the intraocular lens 90.

As illustrated in Fig. 4, the diameter of the plunger 3 is formed to gradually increase from the leading-end side toward the base-end side. Accordingly, at the position where the first restriction member 111 and the second restriction member 112 are provided, as the extrusion of the intraocular lens 90 progresses, the diameter of the plunger 3 gradually increases.

As the diameter of the plunger 3 passing between the first restriction member 111 and the second restriction member 112 increases, the first restriction member 111 and the second restriction member 112 are elastically deformed depending on the shape thereof. Accordingly, the extrusion operation of the intraocular lens 90 is performed in a state in which the contact between the first restriction member 111 and the second restriction member 112 and the plunger 3 is maintained.

The intraocular lens 90 sent from the lens holder 4 to the leading-end tip 5 by the plunger 3 is released into the eye from the release part 52 in a state in which the optical part 91 is folded by the inner wall of the internal passage of the nozzle part 51. Further, the inserting operation of the intraocular lens 90 is appropriately carried out in the state in which the lens holder 4 is filled with a viscoelastic substance such as sodium hyaluronate, hydroxypropylmethyl cellulose, and polyvinyl pyrrolidone, a wound treatment medicine, or the like.

According to the lens holder 4 applied to the intraocular lens injector 1 of the first embodiment described above, the following effects are obtained. The lens holder 4 of the intraocular lens injector 1 according to the first embodiment has a mount unit 62 for setting the optical part 91 of the intraocular lens 90, and includes the holder main body 61 in which the insertion hole 63 of the plunger 3 for extruding the optical part 91 set on the mount unit 62 toward the leading-end side is formed on the base-end side, and the axial-shift-preventing unit 110 as an elastic member that is provided in the movement path of the plunger 3 between the mount unit 62 and the insertion hole 63 inside the holder main body 61, and is deformed in conformance with the shape of the plunger 3 when the plunger 3 moves toward the leading-end side.

As a result, since the axial-shift-preventing unit 110 as the elastic member is deformed with the movement of the plunger 3, it is possible to effectively prevent the shaft shake of the plunger 3 without hindering the movement of the plunger 3. Even when the cross-sectional shape of the plunger 3 increases as it goes from the leading-end side to the base-end side, since the urging force can be continuously exerted by the elastically deforming axial-shift-preventing unit 110, the shaft shake of the plunger 3 can be consistently prevented until the intraocular lens 90 is extruded to the leading-end side of the intraocular lens injector 1.

The shaft-shake-preventing unit 110 has a first restriction member 111 which protrudes from a surface (the side on which the mount unit 62 is disposed) on which the mount unit 62 is disposed in the holder main body 61 and is formed on one side in a direction orthogonal to the movement direction of the plunger 3, and a second restriction member 112 which protrudes form a surface (the side on which the mount unit 62 is disposed) on which the mount unit 62 is disposed in the holder main body 61 and is formed on the other side in a direction orthogonal to the movement direction of the plunger 3. The first restriction member 111 and the second restriction member 112 are formed to approach each other as they are away from the surface on which the mount unit 62 is disposed, and are disposed to sandwich the plunger 3 in the direction orthogonal to the movement direction.

Thus, the force of pressing the plunger 3 against the holder main body 61 side is exerted by the first restriction member 111 and the second restriction member 112, and it is possible to effectively restrict the shifting movement in the left-right direction or the floating movement of the plunger 3.

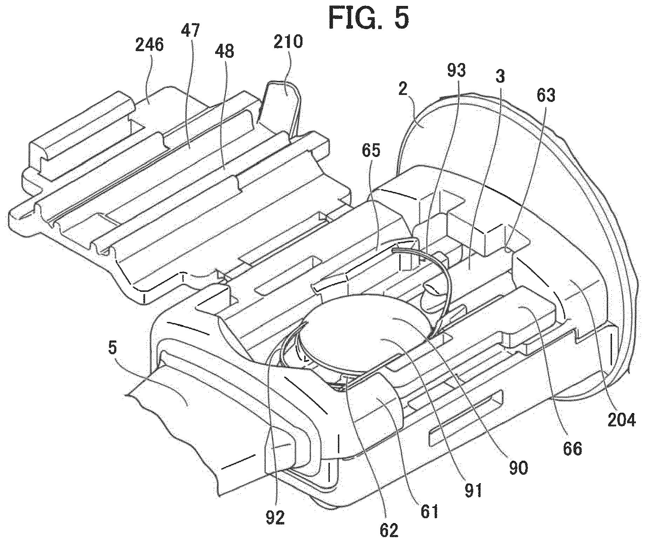

Next, the lens holder 204 of the second embodiment will be described. Fig. 5 is a perspective view illustrating a state in which the intraocular lens 90 is set in the lens holder 204 of the second embodiment.

The lens holder 204 of the second embodiment is different from the lens holder 4 of the first embodiment in the configuration of the axial-shift-preventing unit 110. In the following description, the same configurations as those of the first embodiment are denoted by the same reference numerals, and the description thereof may not be provided.

As illustrated in Fig. 5, in the lens holder 204 of the second embodiment, the axial-shift-preventing unit 210 is formed on the base-end side of the planar part of the cover 246 rather than the holder main body 261. The axial-shift-preventing unit 210 of the second embodiment is integrally formed with a cover 246 to protrude from the inner face of the cover 246. In the axial-shift-preventing unit 210 of the present embodiment, the surface on the side coming into contact with the plunger 3 is curved inward. Further, the axial-shift-preventing unit 210 is not limited to this shape and can be appropriately changed.

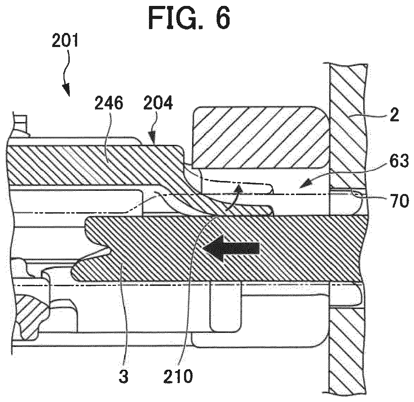

Fig. 6 is a side sectional view illustrating the aspect of the interior of the lens holder 204 of the second embodiment. In Fig. 6, the plunger 3 and the axial-shift-preventing unit 210 after the elastic deformation after movement are illustrated by the chain line.

As illustrated in Fig. 6, in the closed state, the axial-shift-preventing unit 210 is formed to be bent from the cover 246 toward the base-end side of the holder main body 261. The length of the axial-shift-preventing unit 210 is set such that its leading end comes into contact with the upper surface of the plunger 3 in the closed state of the cover 246.

In the state in which the plunger 3 is in contact with the axial-shift-preventing unit 210, the axial-shift-preventing unit 210 is in an elastically deformed state, and a force of pushing the plunger 3 toward the lower part side of the holder main body 261 is exerted. As a result, the movement of the plunger 3 in the upward direction is restricted, and the shaft shake is prevented.

Further, in a natural state that is not in contact with the plunger 3, the position of the axial-shift-preventing unit 210 may be set so that its leading end is located to be closer to the lower part of the holder main body 261 than the position of the upper surface of the leading end part of the plunger 3 at the initial position, the axial-shift-preventing unit 210 may be configured to bias the plunger 3 from the initial position, and the axial-shift-preventing unit 210 may be brought into contact with the plunger 3 after the plunger 3 has moved from the initial position.

When the plunger 3 moves to the leading-end tip 5 side in a state in which the axial-shift-preventing unit 210 is in contact with the plunger 3, since the diameter of the plunger 3 on the base-end side is formed to be larger than that on the leading-end side, as the plunger 3 moves to the leading-end tip 5 side, the axial-shift-preventing unit 210 is pushed upward and elastically deformed (see Fig. 6). Due to the elastic deformation, the contact between the axial-shift-preventing unit 210 and the upper surface of the plunger 3 is maintained, and a force of pushing the plunger 3 to the holder main body 261 side continues to act on the plunger 3.

According to the lens holder 204 applied to the intraocular lens injector 201 of the second embodiment described above, the following effects are obtained. That is, the axial-shift-preventing unit 210 of the lens holder 204 of the second embodiment protrudes from the cover 241, which is a surface on a side opposite to the surface on which the mount unit 62 is disposed in the holder main body 261, and its leading end comes into contact with the plunger 3 in a state of being bent to one side in the movement direction of the plunger 3.

As a result, since the axial-shift-preventing unit 210 is deformed with the movement of the plunger 3, while exerting the urging force on the plunger 3, even in a shape in which the height of the plunger 3 rises toward the base-end side, it is possible to appropriately prevent the shaft shake of the plunger 3, without hindering the movement of the plunger 3.

Further, in the second embodiment, the example in which the axial-shift-preventing unit 210 is formed so as to be bent toward the base-end side in the movement direction has been described, but the configuration is not limited to this example. For example, the axial-shift-preventing unit of the second embodiment may be formed to be bent toward the leading-end side in the movement direction. Also in this case, the axial-shift-preventing unit can prevent the shaft shake of the plunger by the urging force thereof, while being deformed in conformance with the shape of the plunger with the movement of the plunger.

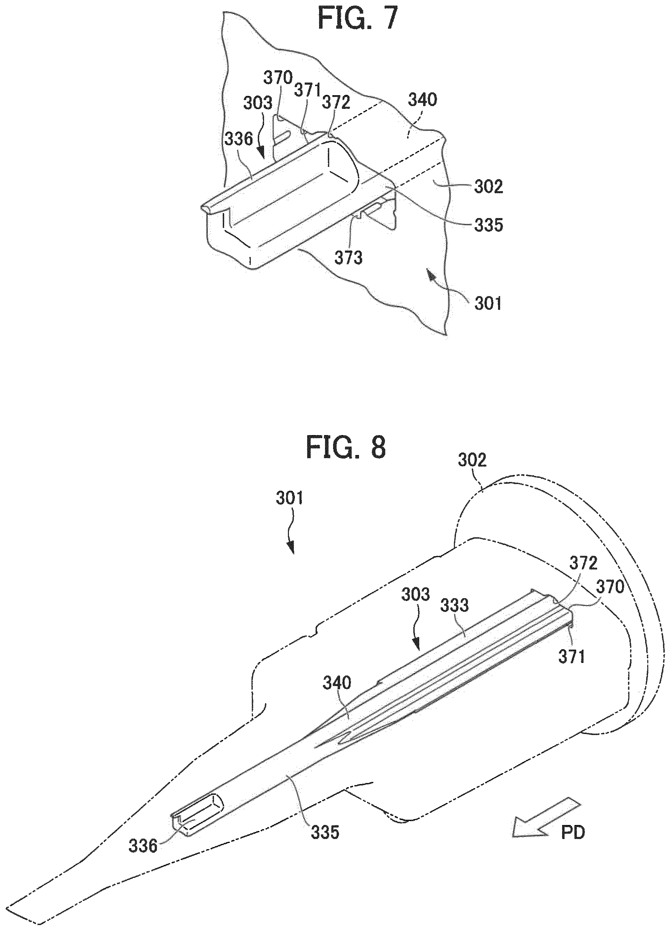

Next, an intraocular lens injector 301 of the third embodiment will be described. Fig. 7 is a perspective view illustrating a positional relation between an opening 370 of a main body 302 of the intraocular lens injector 301 and a plunger 303 at the initial position of the third embodiment. Fig. 8 is a perspective view illustrating an aspect in which the plunger 303 moves from the initial position toward the leading-end side in the intraocular lens injector 301 of the third embodiment.

The intraocular lens injector 301 of the third embodiment is applied to a single piece type intraocular lens. The lens holder 304 of the third embodiment illustrated by the chain line of Fig. 8 is provided with an axial-shift-preventing unit (not illustrated) similar to the axial-shift-preventing unit 110 having the same configuration as the lens holder 4 of the first embodiment or the axial-shift-preventing unit 210 of the second embodiment.

The intraocular lens injector 301 according to the third embodiment is configured to further include, a configuration having a shaft shake preventing function provided by the plunger 303 and the opening 370 formed in the main body 302, in addition to the configuration of the axial-shift-preventing unit provided inside the lens holder 304.

First, the shape of the plunger 303 of the third embodiment will be described. The plunger 303 includes the base-end side shaft part 31 and the pressing part 32 (not illustrated in Figs. 7 and 8), a central-shaft part 333, a leading-end side shaft part 335, an L-shaped section 336, and a restricted part 340.

The base-end side shaft part 31 and the pressing part 32 have the same configuration as the above embodiment (see Fig. 1). The central-shaft part 333 is a shaft part connected to the leading-end side of the base-end side shaft part 31 and having a smaller diameter than the base-end side shaft part 31.

As shown in FIG. 8, the central-shaft part 333 is formed in a shape in which the cross-sectional shape thereof is flat. In addition, the thickness direction of the central-shaft part 333 formed to be flat is the same direction as the thickness direction of the lens holder 304 (vertical direction).

The leading-end side shaft part 335 is connected to the leading-end side of the central-shaft part 333 and is formed to be thinner than the central-shaft part 333. The leading-end side shaft part 335 of the present embodiment has an L-shaped section 336 having an L-shaped cross-sectional shape being in contact with the second lens support part of the intraocular lens at the leading end thereof. In the third embodiment, the lens support part on the leading-end side of the intraocular lens is placed on a notched part of the L-shaped section 336 so that the lens support part can be bent in an appropriate state.

The restricted part 340 is formed along the axial direction of the plunger 303 from the base-end side of the L-shaped section 336 of the leading-end-side shaft part 335 over substantially the entire region of the central-shaft part 333. The restricted part 340 is formed continuously without being interrupted from the leading-end-side shaft part 335 to the central-shaft part 333, and at the central-shaft part 333 formed to be flat, makes a long and narrow ribbed shape projecting from the flat face thereof in the thickness direction (vertical direction). Then, the cross-sectional shape of the restricted part 340 makes a semi-cylindrical shape. The length in the axial direction of this restricted part 340 is set so that shaft shake is prevented by contacting with the opening 370 in the case of the plunger 303 deviating in the up direction in the state of the initial position described later.

In this way, the plunger 303 of the third embodiment is formed so as to become thinner step-wise as approaching the leading-end side from the base-end side. In addition, the plunger 303 is formed in a curved shape in which the top face on the leading-end side thereof is smooth. In the present embodiment, when viewing in the axial direction, the top face of the L-shaped section 336 is formed in a substantially circular arc shape, and the top face of the restricted part 340 formed from the base-end side of the L-shaped section 336 is configured in a smooth chevron shape from the left-right ends in the left-right direction towards a central part. By the top face of a portion entering the interior of the leading-end tip 5 being formed by a smooth curved face in this way, the leading-end side of the plunger 303 fits with the shape of an inner-cavity top part of the leading-end tip 5, whereby a smooth insertion operation is realized. Furthermore, since the top face on the leading-end side of the plunger 303 is formed by a smooth curved face, a situation in which the inner wall top part of the leading-end tip 5 is damaged by the top face on the leading-end side of the plunger 303 in the course of insertion is also prevented.

Next, the opening 370 will be explained. As shown in FIG. 7, the opening 370 includes a central opening 371, restriction part 372, and bottom-side restriction part 373, which are formed based on the shape of the plunger 303.

The central opening 371 is formed in a substantially rectangular shape that is horizontally long. The central opening 371 of the present embodiment is formed according to the cross-sectional shape of the central-shaft part 333 of the plunger 303, and makes an oblong rectangular shape in the left-right direction in which the corners are rounded.

The restriction part 372 is formed in a notched shape so as to project upwards from a center on the top side (side on one side in the thickness direction) of the central opening 371. The restriction part 372 of the present embodiment is formed in a circular arc shape in which the end face thereof matches the cross-sectional shape of the restricted part 340.

The bottom-side restriction part 373 is formed in a notched shape so as to project downwards from the center of a bottom side (side on the other side in the thickness direction) of the central opening 371. The bottom-side restriction part 373 of the present embodiment is formed in an oblong shape that is horizontally long in the left-right direction in which the corners are rounded to match the shape of the bottom of the L-shaped section 336.

Next, the function of shaft shake prevention by the opening 70 during operation of the plunger 303 will be explained. As illustrated in Fig. 7, the initial position of the plunger 303 is in a state in which a part of the leading-end side shaft part 335 is exposed to the interior of the lens holder 304. Further, the L-shaped section 336 of the plunger 303 is located on the side closer to the base-end side than the lens support part on the base-end side of the intraocular lens (not illustrated).

As mentioned above, the restricted part 340 of the plunger 303 has the length thereof set so as to be restricted by the opening 70 at the initial position; therefore, at the initial position, it is a state in which the restriction part 372 of the opening 370 is opposing with the restricted part 340 of the plunger 303.

In addition, at the initial position, the bottom of the L-shaped section 336 of the plunger 303 is in a state caught in the bottom-side restriction part 373 of the opening 370. In other words, in the state in which the plunger 303 is at the initial position, it enters a state in which shaking orthogonal to the axial direction, which is the vertical direction of the plunger 303, is restricted by the restriction part 372 and the bottom-side restriction part 373 of the opening 70.

In a state in which the plunger 303 is at the initial position, when the plunger 303 is pushed from the initial position to the leading-end side in the direction PD by the user, the L-shaped section 336 of the plunger 303 contacts with the second lens support part positioned at the base-end side of the intraocular lens, and enters a state in which the second lens support part is folded to the side of the optical part. In the present embodiment, since the restricted part 340 is formed without being interrupted from the leading-end side shaft part 335 to the central-shaft part 333, shaking in the axial direction of the plunger 303 will be continually restricted from the initial position until the leading-end face of the plunger 303 contacts the second lens support part. Furthermore, since the movement in the left-right direction and down direction of the plunger 303 is restricted by the bottom-side restriction part 373, it becomes possible to appropriately control contact between the plunger 303 and the second lens support part 93 of the intraocular lens 90.

As shown in FIG. 8, when the plunger 303 further advances to the leading-end side, the optical part is folded by the inner wall of the leading-end tip 5, and is inserted into the eye by the release part 52 in this state. The leading-end side shaft part 335 of the plunger 303 is formed to be thinner than the central-shaft part 333, and thus it becomes possible to maintain contact thereof for long until the intraocular lens 90 is discharged to outside, without interfering with the inner wall of the leading-end tip 5. Furthermore, by the bottom of the plunger 303 being continually restricted by the bottom-side restriction part 373, stable movement to the axial direction of the plunger 303 is realized.

The following such effects are exerted according to the intraocular lens injector 301 of the third embodiment explained above. The intraocular lens injector 301 according to the third embodiment includes a main body 302 formed in a tubular shape, a lens holder 304 having the intraocular lens 90 accommodated therein and disposed at the leading end of the main body 302, and a plunger 303 which is inserted into the main body 302 and extrudes the intraocular lens 90 set in the lens holder 304 from the opening 370 formed on the leading-end side of the main body 302 to the leading-end side. The lens holder 304 is provided with an axial-shift-preventing unit having the same configuration as the axial-shift-preventing unit 110 of the first embodiment or the axial-shift-preventing unit 210 of the second embodiment.

As a result, the shaft shake is also prevented by the opening 370 of the main body 302 in conjunction with the shaft shake prevention structure of the lens holder 304, and the shaft shake of the plunger 303 can be complexly prevented.

The plunger 303 of the third embodiment includes: the leading-end side shaft part 335 (leading-end part) having the L-shaped section 336 (contact part) that contacts the intraocular lens 90; the central-shaft part 333 (flat part) that is connected to a base-end side of the leading-end side shaft part 335, and is formed flat to be wider than the leading-end side shaft part 335; and the restricted part 340 that is formed from the leading-end side shaft part 335 to the central-shaft part 333 along the axial direction of the plunger 303, and projects from the flat face at the central-shaft part 333. In addition, the opening 370 in the main body 302 includes a central opening 371 which is opened in accordance with the shape of the central-shaft part 333; and a restriction part 372 which is formed so as to project in the thickness direction of the central-shaft part 333 from the end face of the central opening 371 in accordance with the shaft of the restricted part 340. Then, the restricted part 340 has a length in the axial direction thereof set so that the L-shaped section 336 is restricted by the restriction part 72 from the initial position prior to starting contact with the second lens support part of the intraocular lens until contacting.

In addition, it is possible to form the leading-end side shaft part 335 (leading-end part) to be thin, while raising the rigidity of the plunger 303, by having the central-shaft part 333 (flat part) formed to be flat, as well as possible to reliably prevent shaft shake occurring while the plunger 303 contacts with the intraocular lens 90.

In addition, the plunger 303 further includes the bottom (second restricted part) that projects along the axial direction of the plunger 303 at the flat face on an opposite side from the face of the central-shaft part 333 at which the restricted part 340 is formed. The opening 370 further includes the bottom-side restriction part 373 (second restriction part) that is arranged on the opposite side to the restriction part 372 sandwiching the central opening 371, and is formed in accordance with the shape of the bottom part of the plunger 303, in which the bottom-side restriction part 373 has a length in the axial direction thereof set so that the L-shaped section 336 is restricted by the bottom-side restriction part 373 from the initial position prior to starting contact with the intraocular lens until contacting.

Since it is thereby possible to restrict movement of the plunger 303 in the thickness direction of the flat part by way of the restriction part 372 and bottom-side restriction part 373 (second restriction part), it is possible to more effectively prevent shaft shake of the plunger 303.

The bottom-side restriction part 373 (second restricted part) is formed continuously from the central-shaft part 333 until the leading-end face of the leading-end side shaft part 335 (leading-end face of L-shaped section 336).

From the point of the time in which the plunger 303 is being inserted in the main body 302 to reach the opening 370, since shaft shake of the plunger 303 is prevented by the bottom-side restriction part 373, it is thereby possible to more finely set the initial position of the plunger 303.

In the third embodiment, the shapes of the opening and the plunger are formed in the substantially same shape, but the shapes of the opening and the plunger may be different as long as the shaft shake of the plunger can be prevented by the opening. In this way, the shape of the plunger and the shape of the opening of the third embodiment can be appropriately changed depending on the circumstances.

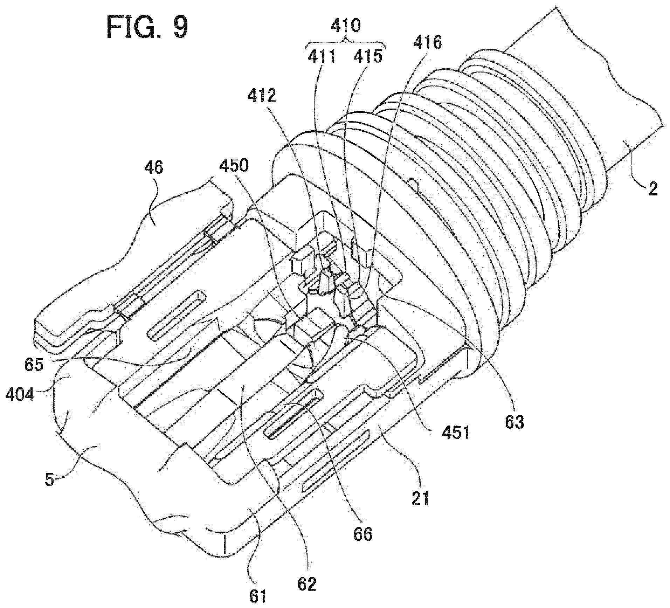

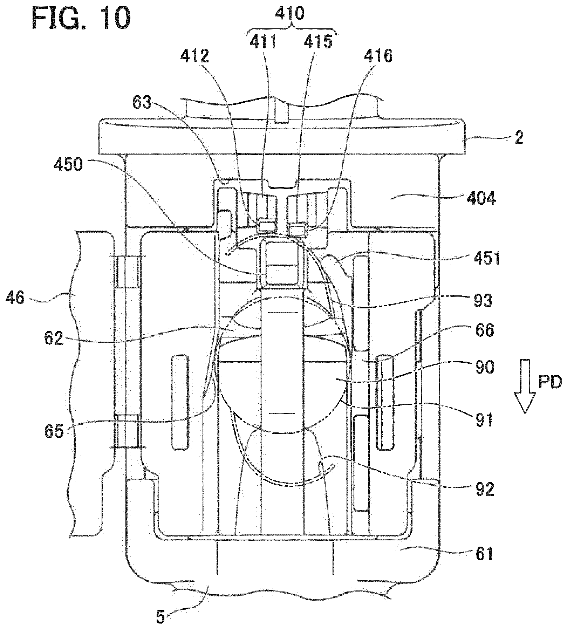

Next, a lens holder 404 of the fourth embodiment will be described. Fig. 9 is a perspective view illustrating an aspect of the interior of the lens holder 404 of the fourth embodiment. Fig. 10 is a plan view illustrating an aspect of the interior of the lens holder 404 of the fourth embodiment.

As illustrated in Figs. 9 and 10, the lens holder 404 according to the fourth embodiment is configured to further include an axial-shift-preventing unit 410 and a support member 450 that are different from the configurations of the above embodiment.

The axial-shift-preventing unit 410 of the fourth embodiment includes a first restriction member 411 and a second restriction member 415. The basic configurations of the first restriction member 411 and the second restriction member 415 themselves are the same as those of the first restriction member 111 and the second restriction member 112 of the first embodiment, except that the first restriction member 411 and the second restriction member 415 include a first wall part 412 and a second wall part 416, respectively.

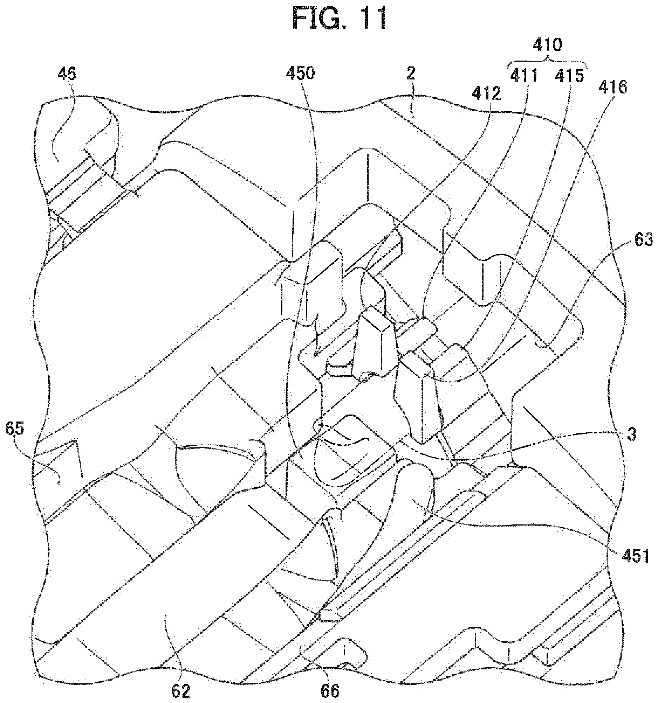

First, a detailed configuration of the axial-shift-preventing unit 410 of the fourth embodiment will be described. Fig. 11 is an enlarged perspective view illustrating the axial-shift-preventing unit 410 and its vicinity of the lens holder of the fourth embodiment. As illustrated in Fig. 11, a wall-shaped first wall part 412 is provided on the leading-end side of the first restriction member 411. The first wall part 412 is configured as a member integral with the first restriction member 411. Similarly, a wall-shaped second wall part 416 is provided integrally with the second restriction member 415 on the leading-end side of the second restriction member 415.

The wall surfaces of the first wall part 412 and the second wall part 416 are oriented toward the leading-end side in the axial direction (traveling direction) of the plunger 3. As illustrated in Fig. 10, the first wall part 412 and the second wall part 416 function as restriction walls which restrict the movement in which the second lens support part 93 located behind the intraocular lens 90 tries to move to the insertion hole 63 side. Therefore, in the intraocular lens 90 before the inserting operation, the shape of the second lens support part 93 can be maintained in an appropriate state, and the inserting operation of the intraocular lens 90 can be performed more smoothly. In addition, it is also possible to prevent the movement of the intraocular lens 90 toward the rear side from the mount unit 62.

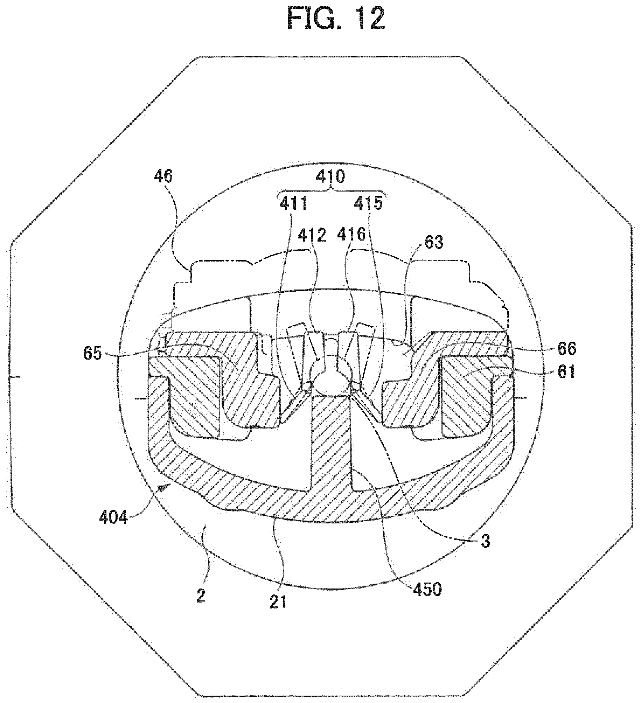

Fig. 12 is a cross-sectional view illustrating the interior of the lens holder 404 of the fourth embodiment as seen from the leading-end tip 5 side. As illustrated in Fig. 12, in the present embodiment, the first wall part 412 is disposed on the upper part of the leading-end side of the first restriction member 411, and the second wall part 416 is disposed on the upper part of the leading-end side of the second restriction member 415. In this way, as viewed in the axial direction, since the first wall part 412 and the second wall part 416 are arranged at positions that do not overlap the locus of movement of the plunger 3, the movement of the plunger 3 is not disturbed.

Further, as illustrated in the plan view of Fig. 10, the positions of the first wall part 412 and the second wall part 416 are offset in the axial direction of the plunger 3. A guide part 451 which guides the direction of the second lens support part 93 is provided on the insertion hole 63 side of the wall part 66 of the fourth embodiment, but the second wall part 416 located on the guide part 451 side is located on the side close to the leading end than the first wall part 412. By adjusting the respective positions of the first wall part 412 and the second wall part 416 in this manner, the position and posture of the second lens support part 93 can be more precisely controlled. In the fourth embodiment, the example in which the first wall part 412 and the second wall part 416 are disposed to be shifted in the axial direction has been described, but the present invention is not limited to this structure. For example, the first wall part 412 and the second wall part 416 can also be disposed at the same position in the axial direction. Further, the first wall part 412 can be located to be closer to the leading-end side than the second wall part 416.

Next, the detailed configuration of the support member 450 will be described. The support member 450 is disposed in the lower part of the lens holder 404. In the present embodiment, the support member 450 is configured to protrude to the cover 46 side of the closed position from the plate-shaped fixing part 21 for fixing the lens holder 404. The height of the upper end surface of the support member 450 is set on the basis of the movement locus of the plunger 3. That is, the support member 450 is provided at a position of being in contact with or close to the lower surface of the plunger 3 in order to restrict the shaft shake of the plunger 3 in the thickness direction of the lens holder 404.

In the fourth embodiment, the force for pressing the plunger 3 against the holder main body 61 side is exerted by the first restriction member 411 and the second restriction member 415, and the downward movement of the plunger 3 is restricted by the support member 450. Thus, shaking of the plunger 3 in the vertical and left/right directions is minimized.

The axial-shift-preventing unit 410 as the elastic member of the fourth embodiment explained above further has a first wall part 412 which is disposed on the leading-end side of the first restriction member 411 and has the surface facing the leading-end side, and a second wall part 416 which is disposed on the leading-end side of the second restriction member 415 and has a surface facing the leading-end side.

Accordingly, the intraocular lens 90 can be set at an appropriate position by the first wall part 412 and the second wall part 416, and the plunger 3 can be brought into contact with the intraocular lens 90 in a more appropriate state. Further, the state of the second lens support part 93 can be more precisely adjusted by the positions of the first wall part 412 and the second wall part 416.

The lens holder 404 according to the fourth embodiment further includes a support member 450 as a lower part side support member which is provided in the movement path of the plunger 3 between the mount unit 62 and the insertion hole 63 and protrudes from the lower part of the lens holder 404 in a direction orthogonal to the movement direction of the plunger 3.

Thus, since the plunger 3 is supported by the support member 450, it is possible to effectively suppress the vertical shaft shake of the plunger 3.

The support member 450 of the fourth embodiment is configured to restrict the shaft shake of the plunger 3 from the fixing part 21 of the main body 2 through the lower part of the lens holder 404 but may be provided at the lower part of the lens holder 404.

The support member 450 according to the fourth embodiment described above can also be provided in the lens holder 4 of the first embodiment, the lens holder 204 of the second embodiment, and the lens holder 304 of the third embodiment.

Although each of the preferred embodiments of an intraocular lens injector of the present invention have been explained above, the present invention is not to be limited to the aforementioned embodiments, and further modifications are possible as appropriate.

Although the intraocular lens injector in which the lens holder and main body are configured separately has been explained as an example in the above-mentioned embodiments, it is not necessarily limited to this configuration, and it is possible to make a configuration in which the lens holder is formed integrally with the main body. In addition, the configuration of the lens holder is not necessarily limited to the configuration of the present embodiment. So long as having the configuration of the present invention that prevents shaft shake of the plunger, the intraocular lens injection can modify the respective configurations such as the main body, leading-end tip, lens holder and plunger thereof. Further, the intraocular lens can also correspond to various shapes such as single piece and three pieces.

### EXPLANATION OF REFERENCE NUMERALS

- 1: Intraocular lens injector
- 2: Main body
- 3: Plunger
- 4, 204, 304, 404: Lens holder
- 90: Intraocular lens
- 91: Optical part (lens main body)
- 61: Holder main body
- 110, 210, 410: Axial-shift-preventing unit (elastic member)
- 111, 411: First restriction member
- 112, 415: Second restriction member
- 333: Central-shaft part (flat part, second restricted part)
- 335: Leading end shaft part (leading-end part)
- 336: L-shaped section 36 (contact part, second restricted part)
- 340: Restricted part
- 370: Opening
- 371: Central opening
- 372: Restriction part
- 373: Lower part side restriction part (second restriction part)
- 412: First wall part
- 416: Second wall part
- 450: Support member (lower part side support member)

## Claims

1. A lens holder (4) of an intraocular lens (90) injector (1) which extrudes the intraocular lens by a plunger (3) and inserts the intraocular lens into an eye, the lens holder comprising:
a holder main body (61) which has a mount unit (62) for setting a lens main body of the intraocular lens and in which an insertion hole (63) of the plunger for extruding the lens main body set on the mount unit to the leading-end side is formed on a base-end side; and
an elastic member (110) which is provided in a movement path of the plunger between the mount unit and the insertion hole inside the holder main body and deforms in conformance with the shape of the plunger when the plunger moves to the leading-end side.

2. The lens holder of the intraocular lens injector according to claim 1, wherein the elastic member has:
a first restriction member which protrudes from a surface of the holder main body on which the mount unit is disposed and is formed on one side in a direction orthogonal to a movement direction of the plunger; and
a second restriction member which protrudes from a surface of the holder main body on which the mount unit is disposed and is formed on the other side in a direction orthogonal to the movement direction of the plunger, and
the first restriction member and the second restriction member are formed to approach each other as the first restriction member and the second restriction member go away from the surface on which the mount unit is disposed, and are disposed to sandwich the plunger in the direction orthogonal to the movement direction.

3. The lens holder of the intraocular lens injector according to claim 2, wherein the elastic member further has:
a first wall part which is disposed on a leading-end side of the first restriction member and has a surface facing the leading-end side; and
a second wall part which is disposed on the leading-end side of the second restriction member and has a surface facing the leading-end side.

4. The lens holder of the intraocular lens injector according to claim 1,
wherein the elastic member protrudes from a surface opposite to the surface of the holder main body on which the mount unit is disposed, and comes into contact with the plunger in a state in which the leading end thereof is bent to one side or the other side in the movement direction of the plunger.

5. The lens holder of the intraocular lens injector according to any one of claims 1 to 4, further comprising:
a lower part side support member which is provided in the movement path of the plunger between the mount unit and the insertion hole, and protrudes from a lower part of the lens holder in a direction orthogonal to the movement direction of the plunger.

6. An intraocular lens injector comprising:
the lens holder according to any one of claims 1 to 5;
a main body having the lens holder disposed at a leading end thereof and formed in a cylindrical shape; and
a plunger which is inserted into the main body and extrudes the intraocular lens set on the lens holder to the leading-end side from an opening formed at the leading-end side of the main body.

7. The intraocular lens injector according to claim 6, wherein the plunger includes:
a leading-end part having a contact part that contacts with the intraocular lens;
a flat part that is connected to a base-end side of the leading-end part, and is formed to be flat and wider than the leading-end part; and
a restricted part that is formed along an axial direction of the plunger from the leading-end part to the flat part, and projects from a flat face at the flat part,
wherein the opening includes:
a central opening that is opened in accordance with the shape of the flat part; and
a restriction part that is formed so as to project in a thickness direction of the flat part from an end face of the central opening in accordance with the shape of the restricted part, and
wherein the restricted part has a length in the axial direction thereof set so as to be restricted by at least the restriction part from an initial position prior to the contact part starting contact with the intraocular lens until contacting.

## Patentansprüche

1. Linsenhalterung (4) eines Injektors (1) einer Intraokularlinse (90), der die Intraokularlinse durch einen Kolben (3) ausstößt und die Intraokularlinse in ein Auge einführt, wobei die Linsenhalterung umfasst:
einen Halterungshauptkörper (61), der eine Befestigungseinheit (62) zum Einrichten eines Linsenhauptkörpers der Intraokularlinse aufweist und in dem ein Einführloch (63) des Kolbens, um den auf der Befestigungseinheit eingerichteten Linsenhauptkörper zu der Vorderendseite auszustoßen, auf einer Basisendseite gebildet ist; und
ein elastisches Element (110), das in einer Bewegungsbahn des Kolbens zwischen der Befestigungseinheit und dem Einführloch innerhalb des Halterungshauptkörpers vorgesehen ist und sich in Übereinstimmung mit der Form des Kolbens verformt, wenn sich der Kolben zu der Vorderendseite bewegt.

2. Linsenhalterung des Injektors einer Intraokularlinse nach Anspruch 1, wobei das elastische Element aufweist:
ein erstes Begrenzungselement, das von einer Oberfläche des Halterungshauptkörpers vorsteht, auf dem die Befestigungseinheit angeordnet ist, und das auf einer Seite in einer Richtung gebildet ist, die orthogonal zu einer Bewegungsrichtung des Kolbens verläuft; und
ein zweites Begrenzungselement, das von einer Oberfläche des Halterungshauptkörpers vorsteht, auf dem die Befestigungseinheit angeordnet ist, und das auf der anderen Seite in einer Richtung gebildet ist, die orthogonal zu der Bewegungsrichtung des Kolbens verläuft, und wobei das erste Begrenzungselement und das zweite Begrenzungselement so gebildet sind, dass sie sich einander nähern, wenn das erste Begrenzungselement und das zweite Begrenzungselement von der Oberfläche weggehen, auf der die Befestigungseinheit angeordnet ist, und so angeordnet sind, dass sie den Kolben in der zu der Bewegungsrichtung orthogonalen Richtung einschichten.

3. Linsenhalterung des Injektors einer Intraokularlinse nach Anspruch 2, wobei das elastische Element ferner aufweist:
einen ersten Wandteil, der auf einer Vorderendseite des ersten Begrenzungselements angeordnet ist und eine Oberfläche aufweist, die der Vorderendseite zugewandt ist; und
einen zweiten Wandteil, der auf einer Vorderendseite des zweiten Begrenzungselements angeordnet ist und eine Oberfläche aufweist, die der Vorderendseite zugewandt ist.

4. Linsenhalterung des Injektors einer Intraokularlinse nach Anspruch 1,
wobei das elastische Element von einer Oberfläche gegenüber der Oberfläche des Halterungshauptkörpers, auf der die Befestigungseinheit angeordnet ist, vorsteht und mit dem Kolben in einem Zustand in Kontakt kommt, in dem sein Vorderende auf eine Seite oder die andere Seite in der Bewegungsrichtung des Kolbens gebogen ist.

5. Linsenhalterung des Injektors einer Intraokularlinse nach einem der Ansprüche 1 bis 4, ferner umfassend:
ein Stützelement auf der Seite eines unteren Teils, das in der Bewegungsbahn des Kolbens zwischen der Befestigungseinheit und dem Einführloch vorgesehen ist und von einem unteren Teil der Linsenhalterung in einer zu der Bewegungsrichtung des Kolbens orthogonalen Richtung vorsteht.

6. Injektor einer Intraokularlinse, umfassend:
die Linsenhalterung nach einem der Ansprüche 1 bis 5;
einen Hauptkörper, der die Linsenhalterung, die an seinem Vorderende angeordnet ist, aufweist und in einer zylindrischen Form gebildet ist; und
einen Kolben, der in den Hauptkörper eingeführt wird und die auf der Linsenhalterung eingerichtete Intraokularlinse zu der Vorderendseite aus einer Öffnung ausstößt, die an der Vorderendseite des Hauptkörpers gebildet ist.

7. Injektor einer Intraokularlinse nach Anspruch 6, wobei der Kolben umfasst:
einen Vorderendteil, der einen Kontaktteil aufweist, der mit der Intraokularlinse in Kontakt tritt;
einen flachen Teil, der mit einer Basisendseite des Vorderendteils verbunden ist und so gebildet ist, dass er flach und breiter ist als der Vorderendteil; und
einen begrenzten Teil, der entlang einer axialen Richtung des Kolbens von dem Vorderendteil zu dem flachen Teil gebildet ist und von einer flachen Fläche an dem flachen Teil vorsteht,
wobei die Öffnung umfasst:
eine zentrale Öffnung, die gemäß der Form des flachen Teils geöffnet ist; und
einen Begrenzungsteil, der so gebildet ist, dass er in einer Dickenrichtung des flachen Teils von einer Endfläche der zentralen Öffnung gemäß der Form des begrenzten Teils vorsteht, und
wobei der begrenzte Teil eine Länge in seiner axialen Richtung aufweist, die so eingerichtet ist, dass er durch mindestens den Begrenzungsteil von einer Ausgangsposition, bevor der Kontaktteil beginnt, mit der Intraokularlinse in Kontakt zu treten, bis zur Kontaktherstellung begrenzt wird.

## Revendications

1. Porte-lentille (4) d'un injecteur (1) destiné à une lentille intraoculaire (90) qui extrude la lentille intraoculaire par un piston (3) et insère la lentille intraoculaire dans un œil, le porte-lentille comprenant :
un corps principal de maintien (61) qui a une unité de montage (62) pour régler un corps principal de lentille de la lentille intraoculaire et dans lequel un trou d'insertion (63) du piston pour extruder le corps principal de lentille réglé sur l'unité de montage jusqu'au côté d'extrémité avant est formé sur un côté d'extrémité de base ; et
un élément élastique (110) qui est placé dans une trajectoire de mouvement du piston entre l'unité de montage et le trou d'insertion à l'intérieur du corps principal de maintien et qui se déforme en conformité avec la forme du piston lorsque le piston se déplace jusqu'au côté d'extrémité avant.

2. Porte-lentille de l'injecteur destiné à une lentille intraoculaire selon la revendication 1, dans lequel l'élément élastique a :
un premier élément de restriction qui fait saillie depuis une surface du corps principal de maintien sur laquelle l'unité de montage est placée et est formée sur un côté dans une direction orthogonale à une direction de mouvement du piston ; et
un second élément de restriction qui fait saillie depuis une surface du corps principal de maintien sur laquelle l'unité de montage est placée et est formée sur l'autre côté dans une direction orthogonale à une direction de mouvement du piston, et le premier élément de restriction et le second élément de restriction sont formés pour s'approcher l'un de l'autre comme le premier élément de restriction et le second élément de restriction s'éloignent de la surface sur laquelle l'unité de montage est placée, et sont placés pour mettre le piston en sandwich dans la direction orthogonale à la direction de mouvement.

3. Porte-lentille de l'injecteur destiné à une lentille intraoculaire selon la revendication 2, dans lequel l'élément élastique a en outre :
une première partie de paroi qui est placée sur un côté d'extrémité avant du premier élément de restriction et a une surface faisant face au côté d'extrémité avant ; et
une seconde partie de paroi qui est placée sur le côté d'extrémité avant du second élément de restriction et a une surface faisant face au côté d'extrémité avant.

4. Porte-lentille de l'injecteur destiné à une lentille intraoculaire selon la revendication 1,
dans lequel l'élément élastique fait saillie depuis une surface opposée à la surface du corps principal de maintien sur laquelle l'unité de montage est placée, et entre en contact avec le piston dans un état dans lequel l'extrémité avant de celui-ci est pliée sur un côté ou l'autre côté dans la direction de mouvement du piston.

5. Porte-lentille de l'injecteur destiné à une lentille intraoculaire selon l'une quelconque des revendications 1 à 4, comprenant en outre :
un élément de maintien latéral d'une partie inférieure qui est placé dans la trajectoire de mouvement du piston entre l'unité de montage et le trou d'insertion et qui fait saillie depuis une partie inférieure du porte-lentille dans une direction orthogonale à la direction de mouvement du piston.

6. Injecteur destiné à une lentille intraoculaire comprenant :
le porte-lentille selon l'une quelconque des revendications 1 à 5 ;
un corps principal ayant le porte-lentille placé à une extrémité avant de celui-ci et formé dans une forme cylindrique ; et
un piston qui est inséré dans le corps principal et qui extrude la lentille intraoculaire placée sur le porte-lentille au côté d'extrémité avant depuis une ouverture formée au côté d'extrémité avant du corps principal.

7. Injecteur destiné à une lentille intraoculaire selon la revendication 6, dans lequel le piston comprend :
une partie d'extrémité avant ayant une partie de contact qui entre en contact avec la lentille intraoculaire ;
une partie plate qui est reliée à un côté d'extrémité de base de la partie d'extrémité avant et qui est formée pour être plate et plus large que la partie d'extrémité avant ; et
une partie restreinte qui est formée le long d'une direction axiale du piston depuis la partie d'extrémité avant jusqu'à la partie plate et qui fait saillie depuis une face plate à la partie plate,
dans lequel l'ouverture comprend :
une ouverture centrale qui est ouverte conformément à la forme de la partie plate ; et
une partie de restriction qui est formée de manière à faire saillie dans une direction de l'épaisseur de la partie plate depuis une face d'extrémité de l'ouverture centrale conformément à la forme de la partie restreinte, et
dans lequel la partie restreinte a une longueur dans la direction axiale de celle-ci réglée de manière à être restreinte par au moins la partie de restriction depuis une position initiale avant que la partie de contact commence à entrer en contact avec la lentille intraoculaire jusqu'au contact.
